# EUROPEAN PATENT APPLICATION

(11) **EP 4 534 550 A1**
(43) Date of publication of application: **09.04.2025**
(21) Application number: 23814743.3
(22) Date of filing: 30.03.2023
(51) Int. Cl.: C07K 14/34, C12N 15/31, C12N 15/77, C12N 1/21, C12P 13/08

(54) **NCGL2747 GENE MUTANT AND USE THEREOF IN PREPARATION OF L-LYSINE**

(30) Priority: 01.06.2022 CN 202210616402
(71) Applicant: Ningxia Eppen Biotech Co., Ltd, Yinchuan, Ningxia 750199 (CN)
(72) Inventor: MENG, Gang, Yinchuan, Ningxia 750199 (CN); ZHOU, Xiaoqun, Yinchuan, Ningxia 750199 (CN); WEI, Aiying, Yinchuan, Ningxia 750199 (CN); ZHAO, Chunguang, Yinchuan, Ningxia 750199 (CN); MA, Fengyong, Yinchuan, Ningxia 750199 (CN); MA, Wenyou, Yinchuan, Ningxia 750199 (CN)
(74) Representative: Winter, Brandl - Partnerschaft mbB
(86) International application number: PCT/CN2023/084970
(87) International publication number: WO 2023/231547

(57) **Abstract**

Provided are an NCgl2747 gene mutant and the use thereof in the preparation of L-lysine. The NCgl2747 gene mutant is a DNA molecule shown in SEQ ID No.3, which encodes the NCgl2747 mutant protein shown in SEQ ID No.4. Mutating the NCgl2747 gene into the NCgl2747 gene mutant shown in SEQ ID No.3 or overexpressing NCgl2747 gene mutant contributes to increase in the yield and the growth rate of L-lysine. However, when the gene is weakened or knocked out, accumulation of the L-lysine is not facilitated, and the growth rate of a strain can be reduced. The NCgl2747 gene mutant and the NCgl2747 mutant protein encoded thereby can be used for preparing L-lysine.

## Description

### Technical Field

The present application relates to an NCgl2747 gene mutant and the use thereof in the preparation of L-lysine in the field of biotechnology.

### Background Art

L-lysine has physiological effects such as promoting development, enhancing immunity, and improving the function of central nervous tissue. It is one of the eight basic amino acids that humans and animals cannot synthesize by themselves and are essential for growth. At present, the L-lysine is the second largest amino acid species in the world, and the main production method is fermentation. *Corynebacterium glutamicum* is an important lysine-producing strain. About 90% of L-lysine's industrial production is used as a nutritional supplement in the feed industry, and 10% thereof is used as a flavoring agent and a sweetener in the food industry, and as a drug intermediate in the pharmaceutical industry.

Improvements in the production of the L-lysine by the fermentation method can involve fermentation technologies such as stirring and oxygen supply; or the composition of the nutrient medium, such as sugar concentration during the fermentation; or the processing of fermentation broth into a suitable product form, e.g., by drying and pelleting the fermentation broth or ion exchange chromatography; or inherent performance properties of microorganisms themselves. Methods used to improve the performance properties of these microorganisms include mutagenesis, and mutant selection and screening. Strains obtained in this way are resistant to antimetabolites or auxotrophic for metabolites of regulatory importance and produce the L-lysine.

### Summary of the Invention

The present invention aims to provide a protein that can produce L-lysine and a related biological material thereof.

In order to solve the above technical problems, the present invention first provides a protein, which is named NCgl2747^{A955T}. NCgl2747^{A955T} is the following A1) or A):
A1) A protein comprising an amino acid sequence of SEQ ID No.4;
A2) A fusion protein obtained by connecting a tag to an N-terminal or/and a C-terminal of A1).

In order to facilitate the purification of the protein in A1), tags as shown in the table below can be connected to an amino terminal or a carboxyl terminal of the protein consisting of the amino acid sequence shown in SEQ ID No.4.

**Table: Sequence of tags**

| Tags | Residue | Sequence |
|---|---|---|
| Poly-Arg | 5-6 (usually 5) | RRRRR |
| Poly-His | 2-10 (usually 6) | HHHHHH |
| FLAG | 8 | DYKDDDDK |
| Strep-tag II | 8 | WSHPQFEK |
| c~myc | 10 | EQKLISEEDL |

The present invention further provides a biological material related to NCgl2747^{A955T}, which is any one of the following B1) to B4):
B1) A nucleic acid molecule encoded as NCgl2747^{A955T};
B2) An expression cassette comprising the nucleic acid molecule in B1);
B3) A recombinant vector comprising the nucleic acid molecule in B1), or a recombinant vector comprising the expression cassette in B2);
B4) A recombinant microorganism comprising the nucleic acid molecule in B1), or a recombinant microorganism comprising the expression cassette in B2), or a recombinant microorganism comprising the recombinant vector in B3).

In the above biological materials, the nucleic acid molecule in B1) may be the following b11) or b12) or b13):
b11) A DNA molecule shown in SEQ ID No.3 in a sequence listing;
b12) A DNA molecule that has 75% or more identity with a nucleotide sequence defined by b11) and is encoded as NCgl2747^{A955T};
b13) A genomic DNA molecule that hybridizes with the nucleotide sequence defined by b11) or b12) under stringent conditions and is encoded as NCgl2747^{A955T}.

Wherein the nucleic acid molecule may be DNA, such as cDNA, genomic DNA or recombinant DNA; the nucleic acid molecule may also be RNA, such as mRNA or hnRNA.

Those ordinary skilled in the art can easily use known methods, such as directed evolution and point mutation, to mutate the nucleotide sequence of the present invention which encodes the NCgl2747^{A955T}; protein. Those nucleotides that have been artificially modified and have 75% or higher identity with the nucleotide sequence of the NCgl2747^{A955T}; protein of the present invention, as long as they encode the NCgl2747^{A955T}; protein and have the function of the NCgl2747^{A955T}; protein, are all derived from the nucleotide sequences of the present invention and are equivalent to the sequences of the present invention.

The term "identity" used herein refers to sequence similarity to a native nucleic acid sequence. The "identity" includes nucleotide sequences having 75% or higher, or 85% or higher, or 90% or higher, or 95% or greater identity with the nucleotide sequence which encodes the protein consisting of the amino acid sequence shown in SEQ ID No.4 in the present invention. The identity may be evaluated with the naked eye or using computer software. Using the computer software, the identity between two or more sequences may be expressed as a percentage (%), which may be used to evaluate the identity between related sequences.

In the above biological materials, the stringent conditions can be as follows: hybridizing in a mixed solution of 7% sodium dodecyl sulfate (SDS), 0.5M NaPO₄ and 1mM EDTA at 50°C, and rinsing in 50°C, 2×SSC, 0.1%SDS; or hybridizing in a mixed solution of 7% SDS, 0.5M NaPO₄ and 1mM EDTA at 50°C, and rinsing in 50°C, 1×SSC, 0.1%SDS; or hybridizing in a mixed solution of 7% SDS, 0.5M NaPO₄ and 1mM EDTA at 50°C, and rinsing in 50°C, 0.5×SSC, 0.1%SDS; or hybridizing in a mixed solution of 7% SDS, 0.5M NaPO₄ and 1mM EDTA at 50°C, and rinsing in 50°C, 0.1×SSC, 0.1%SDS; or hybridizing in a mixed solution of 7% SDS, 0.5M NaPO₄ and 1mM EDTA at 50°C, and rinsing in 65°C, 0.1×SSC, 0.1%SDS; or hybridizing in 6×SSC, 0.5%SDS solution at 65°C, and then washing membrane once with 2×SSC, 0.1% SDS and 1×SSC, 0.1% SDS respectively; or hybridizing and washing the membrane twice in 2×SSC, 0.1%SDS solution at 68°C, 5 min each time, and then hybridizing and washing the membrane twice in 0.5×SSC, 0.1%SDS solution at 68°C, 15 min each time; or hybridizing and washing the membrane in 0.1×SSPE (or 0.1×SSC), 0.1%SDS solution at 65°C.

The above 75% or above identity may be 80%, 85%, 90% or 95% or above identity.

In the above biological materials, the expression cassette (NCgl2747^{A955T} gene expression cassette) comprising the nucleic acid molecule encoding the NCgl2747^{A955T} protein in B2) refers to DNA that can express the NCgl2747^{A955T}; protein in a host cell. The DNA can not only include a promoter that promotes the transcription of the NCgl2747^{A955T}; gene, but also include a terminator that terminates the transcription of the NCgl2747^{A955T}; gene. Furthermore, the expression cassette may also include an enhancer sequence.

In the above biological materials, the expression cassette in B2) may specifically be the DNA molecule shown in SEQ ID No.8.

Existing expression vectors can be used to construct a recombinant vector comprising the NCgl2747^{A955T}; gene expression cassette.

In the above biological materials, the vector may be a plasmid, cosmid, phage or a viral vector. The plasmid may specifically be a pK18mobsacB vector or a pXMJ19 vector.

The recombinant vector in B3) may be a recombinant vector pK18-NCgl2747^{A955T}, pK18-NCgl2747^{A955T}OE or pXMJ19-NCgl2747^{A955T.}

The recombinant vector pK18-NCgl2747^{A955T} is a recombinant vector obtained by replacing the fragment (small fragment) between *Xbal I* and *BamH* I recognition sites of the pK18mobsacB vector with the DNA fragment shown in SEQ ID No.5 in the sequence listing, while keeping other sequences of the pK18mobsacB vector unchanged. The recombinant vector pK18-NCgl2747^{A955T}contains the mutation sites (A-T) of the mutant gene NCgl2747^{A955T}; shown in SEQ ID No.3.

The pK18-NCgl2747^{A955T}OE is a recombinant vector obtained by inserting the expression cassette in B2) between recognition sequences of *Xbal I* and *BamH* I of pK18mobsacB.

The pXMJ19-NCgl2747^{A955T} is a recombinant vector obtained by inserting the expression cassette in B2) into the pXMJ19 vector.

In the above biological materials, the microorganism may be yeast, bacteria, algae or fungi. Wherein, the bacteria may be *Corynebacterium glutamicum, Escherichia coli, Pantoea ananatis*, *Bacillus brevis* or *Brevis lactobacillus.*

In one embodiment of the present invention, the *Corynebacterium glutamicum* is *Corynebacterium glutamicum* YP097158 or *Corynebacterium glutamicum* ATCC13032.

The recombinant microorganism in B4) is a recombinant microorganism obtained by replacing the NCgl2747 gene in the microorganism containing the NCgl2747 gene shown in SEQ ID No.1 with the nucleic acid molecule in B1), or introducing the nucleic acid molecule in B1) into the microorganism and expressing it.

In embodiments of the present invention, the recombinant microorganism is a recombinant strain YPL-NCgl2747-1, a recombinant strain L2747-1, a recombinant strain YPL-NCgl2747-3, a recombinant strain L2747-3, a recombinant strain YPL-NCgl2747-5 or a recombinant strain L2747-5.

The recombinant strain YPL-NCgl2747-1 is a strain obtained by replacing the NCgl2747 gene of *Corynebacterium glutamicum* YP097158 with the NCgl2747^{A955T} gene while keeping other sequences unchanged.

The recombinant strain L2747-1 is a strain obtained by replacing the NCgl2747 gene of ATCC13032 with the NCgl2747^{A955T} gene while keeping other sequences unchanged.

The recombinant strain YPL-NCgl2747-3 is a recombinant strain obtained by replacing a spacer region of an upstream homologous arm NCgl1741 and a downstream homologous arm NCgl1742 in a genome of the *Corynebacterium glutamicum* YP097158 with the NCgl2747^{A955T} gene and a promoter thereof (i.e., positions 1-331 in SEQ ID No.8 in the sequence listing), while keeping other nucleotides in the genome of the *Corynebacterium glutamicum* YP097158 unchanged.

The recombinant strain L2747-3 is a recombinant strain obtained by replacing the spacer region of the upstream homologous arm NCgl1741 and the downstream homologous arm NCgl1742 in a genome of the *Corynebacterium glutamicum* ATCC13032 with the NCgl2747^{A955T} gene and a promoter thereof (i.e., positions 1-331 in SEQ ID No.8 in the sequence listing), while keeping other nucleotides in the genome of the *Corynebacterium glutamicum* ATCC13032 unchanged. The recombinant strain YPL-NCgl2747-5 is a recombinant strain obtained by introducing the pXMJ19-NCgl2747^{A955T} into the *Corynebacterium glutamicum* YP097158.

The recombinant strain L2747-5 is a recombinant strain obtained by introducing the pXMJ19-NCgl2747^{A955T} into the *Corynebacterium glutamicum* ATCC13032.

The present invention further provides a method for preparing L-lysine, which includes: expressing NCgl2747^{A955T} in a recipient biological cell, or increasing the content or activity of the NCgl2747^{A955T} in the recipient biological cell, or increasing the content or activity of the protein shown in SEQ ID No.2 in the recipient biological cell to obtain a recombinant biological cell; and culturing the recombinant biological cell to obtain the L-lysine.

In the above method, the biological cell may be yeast, bacterium, algae, fungi, a plant cell or an animal cell capable of synthesizing the L-lysine.

The bacterium is *Corynebacterium glutamicum,* such as *Corynebacterium glutamicum* YP097158. The bacterium in the present invention includes but not limited to the *Corynebacterium glutamicum.* Any containing the Ncgl2747 gene shown in SEQ ID No.1 in the sequence listing and can synthesize the L-lysine can produce the L-lysine using the NCgl2747 mutant protein shown in SEQ ID No.1 and related biological materials thereof in the present invention. For example, the bacterium may be *Corynebacterium glutamicum, Escherichia coli, Pantoea ananatis, Bacillus brevis* or *Brevis lactobacillus.*

The above method can be realized by introducing an encoding gene of the NCgl2747^{A955T} into the recipient biological cell and expressing it, or introducing an encoding gene of the protein shown in SEQ ID No.2 into the recipient biological cell and expressing it;

Or, the recipient biological cell contains a DNA molecule shown in SEQ ID No.1, and the method is realized by replacing the DNA molecule shown in SEQ ID No.1 in the recipient biological cell with a DNA molecule shown in SEQ ID No.3.

In the above method, the recombinant biological cell may be cultured using a culture medium capable of growing the recombinant biological cell;

And/or, the recombinant biological cell is cultured using conditions that enable the growth of the recombinant biological cell.

The recombinant biological cell may be the recombinant microorganism as described above.

The present invention further provides a product for preparing L-lysine, which comprises (or of which the active ingredient is) NCgl2747^{A955T} or the biological material.

NCgl2747^{A955T} or the biological material of the present invention may be used to produce a variety of products, including but not limited to lysine in the embodiments. The amino acids produced may also be glutamic acid, valine, glycine, alanine, leucine, isoleucine, methionine, proline, tryptophan, serine, tyrosine, cysteine, phenylalanine, asparagine, glutamine, threonine, aspartic acid, arginine and histidine, shikimic acid, protocatechuic acid, succinic acid, a-Ketoglutaric acid, citric acid, ornithine, and citrulline. When producing various target products, the production of the target products can be achieved by placing the NCgl2747^{A955T} of the present invention in a synthesis pathway of the target products.

### Instructions for Preservation of Biological Materials

Classification and naming: *Corynebacterium glutamicum*
Strain number: YP097158
Name of depository authority: China General Microbiological Culture Collection Center
Abbreviation: CGMCC
Address: No.3, No.1, Beichen West Road, Chaoyang District, Beijing 100101, China
Collection date: August 16, 2016
Collection center registration number: CGMCC No.12856

### Detailed Description of the Invention

The present invention will be further described in detail below with reference to specific embodiments. The embodiments given are only for illustrating the present invention and are not intended to limit the scope of the present invention. The embodiments provided below can serve as a guide for those ordinary skilled in the art to make further improvements, and do not limit the present invention in any way.

The experimental methods in the following embodiments are all conventional methods unless otherwise specified. The materials, reagents, instruments, etc. used in the following embodiments can be obtained from commercial sources unless otherwise specified. The quantitative experiments in the following embodiments are repeated three times, and the results are averaged. In the following embodiments, unless otherwise specified, the first position of each nucleotide sequence in the sequence listing is the 5' terminal nucleotide of the corresponding DNA/RNA, and the last position thereof is the 3' terminal nucleotide of the corresponding DNA/RNA.

Embodiment 1. Construction of a recombinant vector containing an NCgl2747 gene coding region with point mutation

Based on the genome sequence of *Corynebacterium glutamicum* ATCC13032 published by NCBI, two pairs of primers for amplifying the NCgl2747 gene coding region were designed and synthesized. The point mutation was introduced into the NCgl2747 gene coding region (SEQ ID No.1) of the *Corynebacterium glutamicum* YP097158 (collection number: CGMCC No.12856, collection date: August 16, 2016, depository authority: Institute of Microbiology, Chinese Academy of Sciences, No.3, No.1, Beichen West Road, Chaoyang District, Beijing, tel.: 010-64807355) and a wild type *Corynebacterium glutamicum* strain ATCC13032. The point mutation refers to mutating the adenine (A) at position 955 in the nucleotide sequence (SEQ ID No.1) of the NCgl2747 gene into thymine (T) to obtain the DNA molecule shown in SEQ ID No.3 (the mutated NCgl2747 gene, recorded as an NCgl2747^{A955T} gene).

Wherein the amino acid sequence which is encoded by the NCgl2747 gene shown in SEQ ID No.1 is SEQ ID No.2.

The amino acid sequence which is encoded by the NCgl2747^{A955T} gene shown in SEQ ID No.3 is a mutant protein (i.e., NCgl2747^{A955T} protein) in SEQ ID No.4. The phenylalanine (F) at position 319 in the amino acid sequence (SEQ ID No.4) of the NCgl2747^{I319F} protein is mutated from the isoleucine (I) at position 319 of the NCgl2747 protein.

The recombinant vector is constructed using an NEBuilder assembly technology. The primers are designed as follows (synthesized by Shanghai Invitrogen Company). The nucleotides in bold font are the mutation positions:
P1:
   5'-CAGTGCCAAGCTTGCATGCCTGCAGGTCGACTCTAGTCACCCGCTACGAAGTTGT-3' (the underlined nucleotide sequence is the sequence on pK18) (SEQ ID No.15),
P2: 5'-CTACCTGAAAAGCGTGCTGAGCTGGGACATTTG-3' (SEQ ID No.16),
P3: 5'-CAAATGTCCCAGCTCAGCACGCTTTTCAGGTAGCTCTCGGTG-3' (SEQ ID No.17),
P4:
   5'-CAGCTATGACCATGATTACGAATTCGAGCTCGGTACCCTTGTTCTACGAATGCCCAC-3' (the underlined nucleotide sequence is the sequence on pK18) (SEQ ID No.18).

Construction method: Using *Corynebacterium glutamicum* ATCC13032 as a template, PCR amplification was performed with primers P1 and P2, P3 and P4 respectively, and two DNA fragments (NCgl2747 Up and NCgl2747 Down) of the NCgl2747 gene coding region, which have mutant nucleotides and sizes of 624bp and 705bp respectively, were obtained.

The above two DNA fragments (NCgl2747 Up and NCgl2747 Down) were separated and purified by agarose gel electrophoresis, and then ligated with the purified pK18mobsacB plasmid (Addgene Company) subject to enzyme digestion (*Xbal* I and *BamH* I) using NEBuilder enzyme (NEB Company) at 50°C for 30 min. The single clone grown after the ligated product was transformed into Escherichia coli DH5a was PCR-identified using primers M13F/M13R (M13F: 5'-TGTAAAACGACGGCCAGT-3' (SEQ ID No.19), M13R: 5'-CAGGAAACAGCTATGACC-3' (SEQ ID No.20)), the plasmid was extracted to obtain a positive recombinant vector with correct sequence, which was recorded as pK18-NCgl2747^{A955T}. The recombinant vector contains a kanamycin resistance marker.

The NCgl2747^{A955T}; Up-Down DNA in the recombinant vector pK18-NCgl2747^{A955T} has a size of 1296 bp (SEQ ID No.5) and contains mutation sites (A-T), which would lead to the mutation of the adenine (A) at position 955 in the NCgl2747 gene coding region in *Corynebacterium glutamicum* YP097158 into thymine (T), ultimately resulting in the change of isoleucine (I) of the encoded protein at position 319 to phenylalanine (F).

The recombinant vector pK18-NCgl2747^{A955T} is a recombinant vector obtained by replacing the fragment (small fragment) between *Xbal* I and *BamH* I recognition sites of the pK18mobsacB vector with the DNA fragment shown in SEQ ID No.5 in the sequence listing, while keeping other sequences of the pK18mobsacB vector unchanged. The recombinant vector pK18-NCgl2747^{A955T}contains the mutation sites (A-T) of the mutant gene NCgl2747^{A955T}; shown in SEQ ID No.3.

### Embodiment 2. Construction of an engineering strain containing a gene NCgl2747^{A955T};

The allelic substitution plasmid (pK18-NCgl2747^{A955T}) in Embodiment 1 was transformed into L-lysine-producing strain *Corynebacterium glutamicum* YP097158 by electric shock (for its construction method, please refer to WO2014121669A1; upon confirmation by sequencing, a wild type NCgl2747 gene coding region was retained on the chromosome of this strain) and the wild type *Corynebacterium glutamicum* strain ATCC13032, and cultured on a solid culture plate containing kanamycin (refer to Table 1 for the composition and culture conditions of culture medium). The single colonies produced by culturing were identified by a primer P1 and a universal primer M13R in Embodiment 1 respectively, and the strain that could amplify a 1384bp band was a positive strain. The positive strain was cultured on a culture medium containing 15% sucrose (the culture medium was obtained by increasing the concentration of sucrose in the culture medium in Table 1 to 15g/L), the single colonies produced by culturing were cultured on a culture medium containing and not containing kanamycin respectively, and grew on the culture medium not containing kanamycin, while the strains that did not grow on the culture medium containing kanamycin was further PCR-amplified using the following primers (synthesized by Shanghai Invitrogen Company):
P5: 5'-TCTATCCAAGGCATACCGC-3' (SEQ ID No.21);
P6: 5'-TCCCATTGGTTTCACACAG-3' (SEQ ID No.22).

The obtained DNA fragment (280bp) was processed (95°C high temperature denaturation for 10 min and rapid ice bath for 5 min) and then subject to Single-Strand Conformation Polymorphis (SSCP) electrophoresis (using the amplified fragment of plasmid pK18-NCgl2747^{A1089C} as a positive control, *Corynebacterium glutamicum* ATCC13032 amplified fragment as a negative control, and water as a blank control). Please refer to Table 2 for the preparation and electrophoresis conditions of PAGE for SSCP electrophoresis. Due to different fragment structures and different electrophoresis positions, strains whose electrophoresis positions are inconsistent with the position of the negative control fragment and consistent with the position of the positive control fragment are strains with successful allelic substitution. The NCgl2747^{A955T}; gene fragment of the positive strain was again PCR-amplified by primers P5/P6, and ligated to a PMD19-T vector for sequencing. Through sequence comparison, the strain with mutation (A-T) in a base sequence was a positive strain with successful allelic substitution. The positive strains obtained from *Corynebacterium glutamicum* YP097158 and wild type *Corynebacterium glutamicum* strain ATCC13032 were named YPL-NCgl2747-1 and L2747-1 respectively.

The recombinant strains YPL-NCgl2747-1 and L2747-1 contain the mutated gene NCgl2747^{A955T}; shown in SEQ ID No.3, and can express the protein shown in SEQ ID No.4. The only difference between the recombinant strain YPL-NCgl2747-1 and the *Corynebacterium glutamicum* YP097158 is that: YPL-NCgl2747-1 replaces the NCgl2747 gene of the *Corynebacterium glutamicum* YP097158 with the NCgl2747^{A955T}; gene while keeping the strains obtained with other sequences unchanged. The only difference between the recombinant strain L2747-1 and ATCC13032 is that: L2747-1 is a strain obtained by replacing the NCgl2747 gene of ATCC13032 with the NCgl2747^{A955T}; gene while keeping other sequences unchanged.

**Table 1 Composition and culture conditions of culture medium**

| | Composition | Content |
|---|---|---|
| Culture medium | Sucrose | 10 g/L |
| | Polypeptone | 10 g/L |
| | Beef extract | 10 g/L |
| | Yeast powder | 5 g/L |
| | Carbamide | 2 g/L |
| | Sodium chloride | 2.5 g/L |
| | Agar powder | 20 g/L |
| | Water | |
| | pH 7.0 | |
| Culture condition | Culture temperature: 32°C | |
| | Culture time: 40 h | |

**Table 2 Preparation and electrophoresis conditions of PAGE for SSCP electrophoresis**

| | Composition | Usage (the concentration of the acrylamide is 8%) |
|---|---|---|
| PAGE | 40% acrylamide | 8 mL |
| | ddH₂O | 26 mL |
| | Glycerin | 4 mL |
| | 10×TBE | 2 mL |
| | TEMED | 40 µL |
| | 10%APS | 600 µL |
| Electrophoresis condition | Placing an electrophoresis tank in ice, and using 1×TBE buffer solution, 120V voltage, and an electrophresis time of 10 h | |

### Embodiment 3. Construction of an engineering strain overexpressing an NCgl2747 gene or an NCgl2747^{A955T}; gene on a genome

Based on the genome sequence of *Corynebacterium glutamicum* ATCC13032 published by NCBI, three pairs of primers for amplifying the fragments of the upstream and downstream homologous arms and the NCgl2747 or an NCgl2747^{A955T}; gene coding region and a promoter region were designed and synthesized. The NCgl2747 or NCgl2747^{A955T}; gene was inserted into the *Corynebacterium glutamicum* YP097158 and the wild type *Corynebacterium glutamicum* ATCC13032.

The primers are designed as follows (synthesized by Shanghai Invitrogen Company):
P7:
   5'-CAGTGCCAAGCTTGCATGCCTGCAGGTCGACTCTAGAATGCGTTCTGGACTGAGG-3' (the underlined nucleotide sequence is the sequence on pK18) (SEQ ID No.23),
P8: 5'-GATTGAGTAGCAAGTGCTTTGGTGCACCGAGAACAGATG-3' (SEQ ID No.24),
P9: 5'-CATCTGTTCTCGGTGCACCAAAGCACTTGCTACTCAATC-3' (SEQ ID No.25),
P10: 5'-GATTTAATTGCGCCATCTGCTACTGCTTGTAAGTGGACAGG-3' (SEQ ID No.26),
P11: 5'-CCTGTCCACTTACAAGCAGTAGCAGATGGCGCAATTAAATC-3' (SEQ ID No.27),
P12:
   5'-CAGCTATGACCATGATTACGAATTCGAGCTCGGTACCCGCTATGACACCTTCAACGG ATC-3 (the underlined nucleotide sequence is the sequence on pK18) (SEQ ID No.28).

Construction method: Using the *Corynebacterium glutamicum* ATCC13032 as a template, PCR amplification was performed with primers P7/P8, P9/P10, and P11/P12 to obtain an upstream homologous arm fragment 763bp (corresponding to a partial coding region of an NCgl1740 gene of the *Corynebacterium glutamicum* ATCC13032 and an NCgl1741 gene and a promoter region thereof, with the sequence shown in SEQ ID No.6), an NCgl2747 gene and a promoter fragment 1645bp thereof (the sequence is shown in SEQ ID No.7), and a downstream homologous arm fragment 596bp (corresponding to a partial coding region of an NCgl1742 gene of the *Corynebacterium glutamicum* ATCC13032, with the sequence shown in SEQ ID No.9). After the PCR reaction, the three fragments amplified from each template were separately recovered by electrophoresis using a column type DNA gel recovery kit. The three recovered fragments were ligated with the purified pK18mobsacB plasmid (Addgene Company) subject to *Xbal I* and *BamH* I enzyme digestion using NEBuilder enzyme (NEB Company) at 50°C for 30 min. The single clone grown after the transformation of the ligated product was PCR-identified using an M13 primer (M13F: 5'-TGTAAAACGGACGGCCAGT-3' (SEQ ID No.19), M13R: 5'-CAGGAAACAGCTATGACC-3' (SEQ ID No.20)) to obtain a positive integration plasmid (recombinant vector). The resulting recombinant vector was pK18-NCgl2747OE, the positive integration plasmid contains a kanamycin resistance marker, and the plasmid integrated into the recombinant on the genome can be obtained through kanamycin screening.

In SEQ ID No.7, the promoters of the NCgl1741 gene are located at positions 1-331, and the NCgl1741 gene is located at positions 332-1645.

Using the *Corynebacterium glutamicum* YPL-NCgl2747-1 as a template, PCR amplification was performed with primers P7/P8, P9/P10, and P11/P12 to obtain an upstream homologous arm fragment 763bp (corresponding to a partial coding region of an NCgl1740 gene of the *Corynebacterium glutamicum* ATCC13032 and an NCgl1741 gene and a promoter region thereof, with the sequence shown in SEQ ID No.6), an NCgl2747^{A955T}; gene and a promoter fragment 1645bp thereof (the sequence is shown in SEQ ID No.8), and a downstream homologous arm fragment 596bp (corresponding to a partial coding region of an NCgl1742 gene of the *Corynebacterium glutamicum* ATCC13032, with the sequence shown in SEQ ID No.9). After the PCR reaction, the three fragments amplified from each template were separately recovered by electrophoresis using a column type DNA gel recovery kit. The three recovered fragments were ligated with the purified pK18mobsacB plasmid (Addgene Company) subject to *Xbal I* and *BamH* I enzyme digestion using NEBuilder enzyme (NEB Company) at 50°C for 30 min. The single clone grown after the transformation of the ligated product was PCR-identified using an M13 primer (M13F: 5'-TGTAAAACGACGGCCAGT-3' (SEQ ID No.19), M13R: 5'-CAGGAAACAGCTATGACC-3' (SEQ ID No.20)) to obtain a positive integration plasmid (recombinant vector). The resulting recombinant vector was pK18-NCgl2747^{A955T}OE, the positive integration plasmid contains a kanamycin resistance marker, and the plasmid integrated into the recombinant on the genome can be obtained through kanamycin screening.

In SEQ ID No.8, the promoters of the NCgl2747^{A955T}; gene are located at positions 1-331, and the NCgl2747^{A955T}; gene is located at positions 332-1645.

The correctly sequenced integration plasmids (pK18-NCgl2747OE, pK18-NCgl2747^{A955T}OE) were electrotransformed into *Corynebacterium glutamicum* strain YP097158 and wild type *Corynebacterium glutamicum* ATCC13032 respectively, and cultured in the culture medium. Please refer to Table 1 for the composition and culture conditions of the culture medium. The single colonies produced by culturing were PCR-identified using primers P13/P14. The PCR-amplified strains containing a fragment with a size of 1959bp (the sequence is shown in SEQ ID No.10) were positive strains, and those that failed to amplify the fragment were original strains. The positive strains were streaked and cultured on a solid culture plate containing 15% sucrose (the culture medium was obtained by increasing the concentration of sucrose in the culture medium in Table 1 to 15g/L), and the single colonies produced by culturing were further PCR-identified using primers P15/P16, and the amplified strains with a size of 1600bp (the sequence is shown in SEQ ID No.11) was positive strains that NCgl2747 or NCgl2747^{A1089C} gene and a promoter thereof were integrated into a spacer region of an upstream homologous arm NCgl1741 and a downstream homologous arm Ncgl1742 in a genome of the *Corynebacterium glutamicum.* Strains obtained by taking *Corynebacterium glutamicum* YP097158 as an original strain were named YPL-NCgl2747-2 (not containing mutation sites) and YPL-NCgl2747-3 (containing mutation sites) respectively, and strains obtained by taking *Corynebacterium glutamicum* ATCC13032 as an original stain were named L2747-2 (not containing mutation sites) and L2747-3 (containing mutation sites) respectively.

The recombinant strain YPL-NCgl2747-2 contains double copies of the NCgl2747 gene shown in SEQ ID No.1. Specifically, the recombinant strain YPL-NCgl2747-2 is a recombinant strain by replacing the spacer region of the upstream homologous arm NCgl1741 and the downstream homologous arm NCgl1742 in the genome of the *Corynebacterium glutamicum* YP097158 with the NCgl2747 gene and the promoter thereof (i.e., positions 1-331 in SEQ ID No.7 in the sequence listing), while keeping other nucleotides in the genome of the *Corynebacterium glutamicum* YP097158 unchanged. The recombinant bacteria containing double copies of the NCgl2747 gene can significantly and stably increase the expression quantity of the NCgl2747 gene.

The recombinant strain L2747-2 contains double copies of the NCgl2747 gene shown in SEQ ID No.1. Specifically, the recombinant strain L2747-2 is a recombinant strain by replacing the spacer region of the upstream homologous arm NCgl1741 and the downstream homologous arm NCgl1742 in the genome of the *Corynebacterium glutamicum* ATCC13032 with the NCgl2747 gene and the promoter thereof (i.e., positions 1-331 in SEQ ID No.7 in the sequence listing), while keeping other nucleotides in the genome of the *Corynebacterium glutamicum* ATCC13032 unchanged. The recombinant bacteria containing double copies of the NCgl2747 gene can significantly and stably increase the expression quantity of the NCgl2747 gene.

The recombinant strain YPL-NCgl2747-3 contains the mutated NCgl2747^{A955T}; gene shown in SEQ ID No.3. Specifically, the recombinant strain YPL-NCgl2747-3 is a recombinant strain by replacing the spacer region of the upstream homologous arm NCgl1741 and the downstream homologous arm NCgl1742 in the genome of the *Corynebacterium glutamicum* YP097158 with the NCgl2747^{A955T}; gene and the promoter thereof (i.e., positions 1-331 in SEQ ID No.8 in the sequence listing), while keeping other nucleotides in the genome of the *Corynebacterium glutamicum* YP097158 unchanged.

The recombinant strain L2747-3 contains the mutated NCgl2747^{A955T}; gene shown in SEQ ID No.3. Specifically, the recombinant strain L2747-3 is a recombinant strain by replacing the spacer region of the upstream homologous arm NCgl1741 and the downstream homologous arm NCgl1742 in the genome of the *Corynebacterium glutamicum* ATCC13032 with the NCgl2747^{A955T}; gene and the promoter thereof (i.e., positions 1-331 in SEQ ID No.8 in the sequence listing), while keeping other nucleotides in the genome of the *Corynebacterium glutamicum* ATCC13032 unchanged.

The PCR-identified primers are as follows:
P13: 5'-TCCAAGGAAGATACACGCC-3' (corresponding to the outside of the upstream homologous arm NCgl1740) (SEQ ID No.29),
P14: 5'-GGTCGTAGATTTCATCGGC-3' (corresponding to the inside of the Ncgl2747 gene) (SEQ ID No.30),
P15: 5'-CAACGGTGTCTCAGAACTAATC-3' (corresponding to the inside of the Ncgl2747 gene) (SEQ ID No.31),
P16: 5'-TGGTCGTTGGAATCTTGC-3' (corresponding to the outside of the downstream homologous arm NCgl1742) (SEQ ID No.32).

### Embodiment 4. Construction of an engineering strain overexpressing an NCgl2747 gene or an NCgl2747^{A955T}; gene on a plasmid

Based on the genome sequence of *Corynebacterium glutamicum* ATCC13032 published by NCBI, a pair of primers for amplifying the NCgl2747 or NCgl2747^{A1089C} gene coding region and a promoter region were designed and synthesized. The primers are designed as follows (synthesized by Shanghai Invitrogen Company):
P17:
   5'-GCTTGCATGCCTGCAGGTCGACTCTAGAGGATCCCCCAAAGCACTTGCTACTCAATC -3' (the underlined nucleotide sequence is the sequence on pXMJ19) (SEQ ID No.33),
P18:
   5'-ATCAGGCTGAAAATCTTCTCTCATCCGCCAAAACCTACTGCTTGTAAGTGGACAGG-3 ' (the underlined nucleotide sequence is the sequence on pXMJ19) (SEQ ID No.34).

Construction method: Using *Corynebacterium glutamicum* ATCC13032 and YPL-NCgl2747-1 as templates respectively, PCR amplification was performed with primers P17/P18 to obtain the NCgl2747 gene and a promoter fragment thereof (the sequence is shown in SEQ ID No.12) and the NCgl2747^{A955T} gene and a promoter fragment thereof 1715bp (the sequence is shown in SEQ ID No. 13). The amplified product was electrophoresed and purified and recovered using a column type DNA gel recovery kit. The recovered DNA fragments were ligated with a shuttle plasmid pXMJ19 recovered through EcoR I enzyme digestion using NEBuilder enzyme (NEB Company) at 50°C for 30 min. The single clone grown after the transformation of the ligated product was PCR-identified using a primer M13 to obtain the positive overexpression plasmid pXMJ19-NCgl2747 (containing the NCgl2747 gene) and pXMJ19-NCgl2747^{A955T} (containing the NCgl2747^{A955T}; gene), and the positive plasmid was sent for sequencing. Because the plasmid contains a chloramphenicol resistance marker, chloramphenicol can be used to screen whether the plasmid has been transformed into the strain.

In SEQ ID No.12, the promoters of the NCgl2747 gene are located at positions 37-367, and the NCgl2747 gene is located at positions 368-1681.

In SEQ ID No.13, the promoters of the NCgl2747^{A955T}; gene are located at positions 37-367, and the NCgl2747^{A955T}; gene is located at positions 368-1681.

The correctly sequenced pXMJ19-NCgl2747 and pXMJ19-NCgl2747^{A955T} plasmids were electrotransformed into *Corynebacterium glutamicum* YP097158 and wild type *Corynebacterium glutamicum* ATCC13032, respectively, and cultured in the culture medium. Please refer to Table 1 for the composition and culture conditions of the culture medium. The single colonies produced by culturing were PCR-identified using primers M13R(-48)/P18 (M13R(-48): AGCGGATAACAATTTCACACAGGA). The strains obtained by taking the *Corynebacterium glutamicum* YP097158 as an original strain were named YPL-NCgl2747-4 (containing plasmid pXMJ19-NCgl2747) and YPL-NCgl2747-5 (containing plasmid pXMJ19-NCgl2747^{A955T}) respectively. The strains obtained by taking the *Corynebacterium glutamicum* ATCC13032 as the initial strains were named L2747-4 (containing plasmid pXMJ19-NCgl2747) and L2747-5 (containing plasmid pXMJ19-NCgl2747^{A955T}) respectively.

The recombinant strains YPL-NCgl2747-4 and L2747-4 contain the plasmid carrying the NCgl2747 gene shown in SEQ ID No.1;

The recombinant strains YPL-NCgl2747-5 and L2747-5 contain the plasmid carrying the mutated NCgl2747^{A955T} gene shown in SEQ ID No.3.

### Embodiment 5. Construction of an engineering strain lacking an NCgl2747 gene on a genome

Based on the genome sequence of *Corynebacterium glutamicum* ATCC13032 published by NCBI, two pairs of primers for amplifying the fragments at both ends of the NCgl2747 gene coding region were synthesized and used as fragments of the upstream and downstream homologous arms. The primers are designed as follows (synthesized by Shanghai Invitrogen Company):
P19:
   5'-CAGTGCCAAGCTTGCATGCCTGCAGGTCGACTCTAGGCAGCAAGTAGCCTGGGTAT-3' (the underlined nucleotide sequence is the sequence on pK18) (SEQ ID No.35),
P20: 5'-AGAAGATGAAGGACGTGGGTAACTTCCTGTCCACT-3' (SEQ ID No.36),
P21: 5'-AGTGGACAGGAAGTTACCCACGTCCTTCATCTTCTCCGAC-3' (SEQ ID No.37),
P22:
   5'-CAGCTATGACCATGATTACGAATTCGAGCTCGGTACCCACGCAACACTTGATGGAGT-3' (the underlined nucleotide sequence is the sequence on pK18) (SEQ ID No.38).

Construction method: Using *Corynebacterium glutamicum* ATCC13032 as a template, PCR amplification was performed with primers P19/P20 and P21/P22 respectively, to obtain an upstream homologous arm fragment 647bp and a downstream homologous arm fragment 753bp of the knockout Ncgl2747. The amplified product was electrophoresed and purified using a column DNA gel recovery kit. The recovered DNA fragment was ligated with the purified pK18mobsacB plasmid (Addgene Company) subject to Xbal I/BamH I enzyme digestion using NEBuilder enzyme (NEB Company) at 50°C for 30 min. The single clone grown after the transformation of the ligated product was PCR-identified using a primer M13 to obtain the positive knockout vector pK18-ΔNCgl2747. The plasmid contains the homologous arm fragment 1400bp of the entire knockout Ncgl2747 (the sequence is shown in SEQ ID No.14) and kanamycin resistance as a selection marker, and the plasmid was sent for sequencing.

The correctly sequenced knockout plasmid pK18-ΔNCgl2747 was electrotransformed into *Corynebacterium glutamicum* YP097158 and wild type *Corynebacterium glutamicum* ATCC13032, and cultured in the culture medium. Please refer to Table 1 for the composition and culture conditions of the culture medium. The single colonies produced by culturing were PCR-identified using primers P19/P22: strains that simultaneously amplified 1400bp and 2549bp bands were positive strains, and strains that only amplified 2549bp bands were original strains. The positive strains were screened on a 15% sucrose solid medium and then cultured on a culture medium containing kanamycin and not containing kanamycin respectively. The strains were selected to grow on the culture medium not containing kanamycin, while the strains that did not grow on the culture medium containing kanamycin were further PCR-identified using primers P19/P22, and the strains that amplified the 1400 bp bands were positive strains with the NCgl2747 gene coding region knocked out. The NCgl2747 fragment of the positive strain was again PCR-amplified using the primers P19/P22 and ligated to the pMD19-T vector for sequencing. The correctly sequenced strains were named YPL-NCgl2747-6 (the NCgl2747 gene on the genome of the *Corynebacterium glutamicum* YP097158 was knocked out) and L2747-6 (the NCgl2747 gene on the genome of the wild type *Corynebacterium glutamicum* ATCC13032 was knocked out).

### Embodiment 6. L-lysine fermentation experiment

The strains constructed in Embodiments 1-5 and the original strains of the *Corynebacterium glutamicum* YP097158 and ATCC13032 were subject to a fermentation experiment in a culture medium shown in Table 3 with a control process shown in Table 4 in a BLBIO-5GC-4-H fermentation tank (Shanghai Bailun Biological Technology Co., Ltd.). After the fermentation, the ninhydrin colorimetry method was used to detect the L-lysine yield, and the dissolved oxygen (DO) (660nm) was measured with a spectrophotometer. Each strain was repeated three times, and the results are shown in Table 5.

**Table 3 Fermentation medium formula**

| Composition | Formula |
|---|---|
| Amylolytic sugar | 30 g/L |
| Ammonium sulfate | 12 g/L |
| Magnesium sulfate | 0.87 g/L |
| Molasses | 20 g/L |
| Acidified corn steep liquor | 3 mL/L |
| Phosphoric acid | 0.4 mL/L |
| Potassium chloride | 0.53 g/L |
| Defoamer (2%) | 4 mL/L |
| Ferrous sulfate | 120 mg/L |
| Manganese sulfate | 120 mg/L |
| Nicotinamide | 42 mg/L |
| Calcium pantothenate | 6.3 mg/L |
| Vitamin B1 | 6.3 mg/L |
| Copper and zinc salt solution | 0.6 g/L |
| Biotin | 0.88 mg/L |

**Table 4 Fermentation control process**

| | | | |
|---|---|---|---|
| Correction DO100% | Temperature 37°C, air volume 4L/min, rotating speed 1000 rpm, tank pressure 0 mpa, and calibration for 5 min | | |
| Inoculation amount | 10% | Culture temperature (°C) | 37°C |
| pH | pH6.9±0.05 | DO | 10-30% |
| Initial condition | Temperature 37°C, pH 6.9, tank pressure 0 Mpa, air volume 3 L/min, and rotating speed 550 rpm | | |
| Whole process control | Whole process control: 1. When DO<30%, increasing the rotating speed 750rpm→800rpm→air volume 4L/min→850rpm→950rpm successively; 2. Fermenting for 6 h at the tank pressure of 0.01 Mpa; fermenting for 12 h at the tank pressure of 0.02Mpa→0.03Mpa→0.04Mpa→0.05Mpa | | |
| Residual sugar control | 0.1-0.2% before F12h; controlling residual sugar at 0.1-0.05% after F12h according to DO requirements | | |
| Ammonia nitrogen control | 0.1-0.15 before F12h; 0.15-0.25 between F12-F32h; 0.1-0.15 after F32h | | |
| Materials added | 25% ammonia water, 70% concentrated sugar, 50% ammonium sulfide, 10% defoamer | | |
| Fermentation period | About 48 h | | |

**Table 5 Experimental results of L-lysine fermentation**

| Strain | L-lysine yield (g/100ml) | 0D (660 nm) | Analysis on significant difference of L-lysine yield | |
|---|---|---|---|---|
| YP097158 | 18.9 | 37.3 | | |
| YPL-NCg12747-1 | 19.5 | 38.1 | P<0.05 | Compared with YP097158 |
| YPL-NCg12747-2 | 19.2 | 37.8 | P>0.05 | |
| YPL-NCg12747-3 | 19.7 | 38.2 | P<0.01 | |
| YPL-NCg12747-4 | 19.4 | 38.2 | P<0.05 | |
| YPL-NCg12747-5 | 19.9 | 38.3 | P<0.01 | |
| YPL-NCg12747-6 | 18.0 | 36.8 | P<0.01 | |
| ATCC13032 | 0.20 | 100.2 | | |
| L2747-1 | 0.35 | 92.3 | P<0.01 | Compared with ATCC13032 |
| L2747-2 | 0.36 | 90.2 | P<0.01 | |
| L2747-3 | 0.42 | 91.5 | P<0.01 | |
| L2747-4 | 0.46 | 89.2 | P<0.01 | |
| L2747-5 | 0.48 | 90.9 | P<0.01 | |
| L2747-6 | 0.15 | 98.5 | P<0.01 | |

The results are shown in Table 5. Point mutation NCgl2747^{I319F} and overexpression of the Ncgl2747 gene coding region in the *Corynebacterium glutamicum* contributes to increase in the yield and the growth rate of L-lysine. However, when the gene is weakened or knocked out, accumulation of the L-lysine is not facilitated, and the growth rate of a strain can be reduced.

The present invention is described in detail above. For those skilled in the art, the present invention can be implemented in a wider range under equivalent parameters, concentrations and conditions without departing from the purpose and scope of the present invention and performing unnecessary experiments. Although special embodiments of the present invention have been shown, it should be understood that further improvements can be made to the present invention. In short, based on the principles of the present invention, this application is intended to include any changes in, uses of, or improvements to the present invention, including changes that depart from the scope disclosed in this application and are made using conventional technologies known in the art. Some essential features may be applied within the scope of the appended claims below.

The sequences 1-14 involved in the above embodiments are as follows:
SEQ ID No.1: Wild type ORF (CDS) sequence of NCgl2747 gene (nucleotide sequence 1314bp) 
GTGACTACAGACAAGCGCAAAACCTCTAAGACCACCGACACCGCCAACAAGGCTGTGGGCGCGGATCAGGCAGC
GCGTCCCACTCGGCGAACAACTCGCCGCATCTTCGATCAGTCGGAGAAGATGAAGGACGTGCTGTACGAGATCC
GTGGCCCGGTGGCCGCGGAGGCGGAACGCATGGAGCTTGATGGGCATAACATCTTAAAGCTCAACACGGGAAAT
CCAGCCGTGTTCGGATTCGATGCCCCCGACGTGATTATGCGTGACATGATCGCCAACCTTCCAACTTCCCAAGG
GTATTCCACCTCCAAAGGCATTATTCCGGCCCGGCGAGCAGTGGTCACCCGCTACGAAGTTGTGCCCGGATTCC
CCCACTTCGATGTTGATGATGTGTTCTTAGGCAACGGTGTCTCAGAACTAATCACCATGACCACCCAAGCACTC
CTCAACGACGGCGATGAAGTTCTTATCCCCGCACCGGACTACCCACTGTGGACTGCCGCAACCTCCCTGGCTGG
TGGTAAGCCTGTGCACTACCTCTGTGATGAGGAAGATGACTGGAACCCATCCATCGAAGACATCAAGTCCAAAA
TCTCAGAGAAAACCAAAGCTATTGTGGTGATCAACCCCAACAACCCCACGGGAGCTGTCTACCCGCGCCGGGTG
TTGGAACAAATCGTCGAGATTGCACGCGAGCATGACCTGCTGATTTTGGCCGATGAAATCTACGACCGCATTCT
CTACGATGATGCCGAGCACATCAGCCTGGCAACCCTTGCACCAGATCTCCTTTGCATCACATACAACGGTCTAT
CCAAGGCATACCGCGTCGCAGGATACCGAGCTGGCTGGATGGTATTGACTGGACCAAAGCAATACGCACGTGGA
TTTATTGAGGGCCTCGAACTCCTCGCAGGCACTCGACTCTGCCCAAATGTCCCAGCTCAGCACGCTATTCAGGT
AGCTCTCGGTGGACGCCAGTCCATCTACGACCTCACTGGCGAACACGGCCGACTCCTGGAACAGCGCAACATGG
CATGGACGAAACTCAACGAAATCCCAGGTGTCAGCTGTGTGAAACCAATGGGAGCTCTATACGCGTTCCCCAAG
CTCGACCCCAACGTGTACGAAATCCACGACGACACCCAACTCATGCTGGATCTTCTCCGTGCCGAGAAAATCCT
CATGGTTCAGGGCACTGGCTTCAACTGGCCACATCACGATCACTTCCGAGTGGTCACCCTGCCATGGGCATCCC
AGTTGGAAAACGCAATTGAGCGCCTGGGTAACTTCCTGTCCACTTACAAGCAGTAG
SEQ ID No.2: NCgl2747 protein sequence (i.e., amino acid sequence 438aa encoded by sequence 1) 
MTTDKRKTSKTTDTANKAVGADQAARPTRRTTRRIFDQSEKMKDVLYEIRGPVAAEAERMELDGHNILKLNTGN
PAVFGFDAPDVIMRDMIANLPTSQGYSTSKGIIPARRAVVTRYEVVPGFPHFDVDDVFLGNGVSELITMTTQAL
LNDGDEVLIPAPDYPLWTAATSLAGGKPVHYLCDEEDDWNPSIEDIKSKISEKTKAIVVINPNNPTGAVYPRRV
LEQIVEIAREHDLLILADEIYDRILYDDAEHISLATLAPDLLCITYNGLSKAYRVAGYRAGWMVLTGPKQYARG
FIEGLELLAGTRLCPNVPAQHAIQVALGGRQSIYDLTGEHGRLLEQRNMAWTKLNEIPGVSCVKPMGALYAFPK
LDPNVYEIHDDTQLMLDLLRAEKILMVQGTGFNWPHHDHFRVVTLPWASQLENAIERLGNFLSTYKQ
SEQ ID No.3: Gene mutant NCgl2747^{A955T}ORF (CDS) sequence (nucleotide sequence 1437bp) 
GTGACTACAGACAAGCGCAAAACCTCTAAGACCACCGACACCGCCAACAAGGCTGTGGGCGCGGATCAGGCAGC
GCGTCCCACTCGGCGAACAACTCGCCGCATCTTCGATCAGTCGGAGAAGATGAAGGACGTGCTGTACGAGATCC
GTGGCCCGGTGGCCGCGGAGGCGGAACGCATGGAGCTTGATGGGCATAACATCTTAAAGCTCAACACGGGAAAT
CCAGCCGTGTTCGGATTCGATGCCCCCGACGTGATTATGCGTGACATGATCGCCAACCTTCCAACTTCCCAAGG
GTATTCCACCTCCAAAGGCATTATTCCGGCCCGGCGAGCAGTGGTCACCCGCTACGAAGTTGTGCCCGGATTCC
CCCACTTCGATGTTGATGATGTGTTCTTAGGCAACGGTGTCTCAGAACTAATCACCATGACCACCCAAGCACTC
CTCAACGACGGCGATGAAGTTCTTATCCCCGCACCGGACTACCCACTGTGGACTGCCGCAACCTCCCTGGCTGG
TGGTAAGCCTGTGCACTACCTCTGTGATGAGGAAGATGACTGGAACCCATCCATCGAAGACATCAAGTCCAAAA
TCTCAGAGAAAACCAAAGCTATTGTGGTGATCAACCCCAACAACCCCACGGGAGCTGTCTACCCGCGCCGGGTG
TTGGAACAAATCGTCGAGATTGCACGCGAGCATGACCTGCTGATTTTGGCCGATGAAATCTACGACCGCATTCT
CTACGATGATGCCGAGCACATCAGCCTGGCAACCCTTGCACCAGATCTCCTTTGCATCACATACAACGGTCTAT
CCAAGGCATACCGCGTCGCAGGATACCGAGCTGGCTGGATGGTATTGACTGGACCAAAGCAATACGCACGTGGA
TTTATTGAGGGCCTCGAACTCCTCGCAGGCACTCGACTCTGCCCAAATGTCCCAGCTCAGCACGCTTTTCAGGT
AGCTCTCGGTGGACGCCAGTCCATCTACGACCTCACTGGCGAACACGGCCGACTCCTGGAACAGCGCAACATGG
CATGGACGAAACTCAACGAAATCCCAGGTGTCAGCTGTGTGAAACCAATGGGAGCTCTATACGCGTTCCCCAAG
CTCGACCCCAACGTGTACGAAATCCACGACGACACCCAACTCATGCTGGATCTTCTCCGTGCCGAGAAAATCCT
CATGGTTCAGGGCACTGGCTTCAACTGGCCACATCACGATCACTTCCGAGTGGTCACCCTGCCATGGGCATCCC
AGTTGGAAAACGCAATTGAGCGCCTGGGTAACTTCCTGTCCACTTACAAGCAGTAG
SEQ ID No.4: Gene mutant NCgl2747^{I319F} protein sequence (i.e., amino acid sequence 479aa encoded by sequence 3) 
MTTDKRKTSKTTDTANKAVGADQAARPTRRTTRRIFDQSEKMKDVLYEIRGPVAAEAERMELDGHNILKLNTGN
PAVFGFDAPDVIMRDMIANLPTSQGYSTSKGIIPARRAVVTRYEVVPGFPHFDVDDVFLGNGVSELITMTTQAL
LNDGDEVLIPAPDYPLWTAATSLAGGKPVHYLCDEEDDWNPSIEDIKSKISEKTKAIVVINPNNPTGAVYPRRV
LEQIVEIAREHDLLILADEIYDRILYDDAEHISLATLAPDLLCITYNGLSKAYRVAGYRAGWMVLTGPKQYARG
FIEGLELLAGTRLCPNVPAQHAFQVALGGRQSIYDLTGEHGRLLEQRNMAWTKLNEIPGVSCVKPMGALYAFPK
LDPNVYEIHDDTQLMLDLLRAEKILMVQGTGFNWPHHDHFRVVTLPWASQLENAIERLGNFLSTYKQ
SEQ ID No.5: NCg12747^{A955T}Up_Down (size of 1296bp) 
TCACCCGCTACGAAGTTGTGCCCGGATTCCCCCACTTCGATGTTGATGATGTGTTCTTAGGCAACGGTGTCTCA
GAACTAATCACCATGACCACCCAAGCACTCCTCAACGACGGCGATGAAGTTCTTATCCCCGCACCGGACTACCC
ACTGTGGACTGCCGCAACCTCCCTGGCTGGTGGTAAGCCTGTGCACTACCTCTGTGATGAGGAAGATGACTGGA
ACCCATCCATCGAAGACATCAAGTCCAAAATCTCAGAGAAAACCAAAGCTATTGTGGTGATCAACCCCAACAAC

CCCACGGGAGCTGTCTACCCGCGCCGGGTGTTGGAACAAATCGTCGAGATTGCACGCGAGCATGACCTGCTGAT
TTTGGCCGATGAAATCTACGACCGCATTCTCTACGATGATGCCGAGCACATCAGCCTGGCAACCCTTGCACCAG
ATCTCCTTTGCATCACATACAACGGTCTATCCAAGGCATACCGCGTCGCAGGATACCGAGCTGGCTGGATGGTA
TTGACTGGACCAAAGCAATACGCACGTGGATTTATTGAGGGCCTCGAACTCCTCGCAGGCACTCGACTCTGCCC
AAATGTCCCAGCTCAGCACGCTTTTCAGGTAGCTCTCGGTGGACGCCAGTCCATCTACGACCTCACTGGCGAAC
ACGGCCGACTCCTGGAACAGCGCAACATGGCATGGACGAAACTCAACGAAATCCCAGGTGTCAGCTGTGTGAAA
CCAATGGGAGCTCTATACGCGTTCCCCAAGCTCGACCCCAACGTGTACGAAATCCACGACGACACCCAACTCAT
GCTGGATCTTCTCCGTGCCGAGAAAATCCTCATGGTTCAGGGCACTGGCTTCAACTGGCCACATCACGATCACT
TCCGAGTGGTCACCCTGCCATGGGCATCCCAGTTGGAAAACGCAATTGAGCGCCTGGGTAACTTCCTGTCCACT
TACAAGCAGTAGTAGTTGTTAGGATTCACCACGAATCTCAGGATTTTTGAGATTCGTGGTGAATTTTTGCGTTT
TCCAGTCAGGCTCCTGCAACTTTCGGACCGATTTCAGAGGGGCGGAGCTGGTTTGTGGTGGATCCTTGAAATGG
AACCTCGCAGGAAGCTTTCAGGAAGACCAAGTTGGGCCTAGGGGTGGCGGGATTGCAAAAATCCGTCCCCGGTT
CGCCATGAAATGCTGATTTTGATCGAATCTTTGCGCTAACTGTAGGGCGGGTTCAGGGGGTGAATGCACCACGA
GCAACCCGAAGGGTGCGAAGTGGGCATTCGTAGAACAA
SEQ ID No.6: Upstream homologous arm sequence of genome-integrated P7/P8 NCgl2747 (763bp) 
AATGCGTTCTGGACTGAGGTGACCACCATGCAAGACGAGGTGGACCTAGTGATCACCAACCCGCCGTTTTCTCT
GTTCCGTGAGTTCCTGAGTTGGCTATTACACGGTGACGTGTTGTTTTCTATCATCGGTAACGCGAACGTAATCA
CATATCTAGGTGCCTGAATCTAGATTAAAACTATAAACATTATTTAAAACCATTGCCTTATTGGAGCATGCTGC
AAGCTTTTCGCGGTGGGCTTGCAACATCTTACATCAGAATGAATGATGTTAAACACCTAATAAGTTCAAGTAGT
TAAAAGGAAATATCAACAATGTTACGAAAAGCATCTATCACGCTAATGATATCCGTTACGCTCTTAACATGTGC
CTCTCCAGCACAGGCACTGTCATCACAAGCACTCTCGTCAGAAAGCTCCACTTCGCAGAGTGATTCTCCTACGC
AATTTGTTGCGAGTATAGCTGCGCCCTTCAATAAGAACTTAACCTATGAGCAGCGCAAAGCCATTCGTTTCGGA
CCTACGACAGAACAAGAAGCAGAACAATGCTTGGCAAAGTATGGTCGCGATGGCGAGGGGCCATGGCCTGCTAT
TGCTATCTATCCTGACTGCTCCTTCGATACTATCGAACAAGATTTCTATGATCAAGCAATATCCAAAGCCCTCT
CAGTAATCAGTCTGGGAAGCTCCTAGAAGACTCGTAACGACCAAAAGCACCGAGAACAGATGCAACTCAAGCCC
ATTTACATCTGTTCTCGGTGCAC
SEQ ID No.7: Genome-integrated P9/P10 NCgl2747 and promoter sequence thereof (1645bp) 
CAAAGCACTTGCTACTCAATCATGGTAAACAGCCTGGTCGCAGTCCTTCACGATTCAAACTTTGCCTTCCGCTA
CGCCTTCCACCTGATCATCATAGAAGACGGTGAAGTAACAGCAGCCGGAGATCCCACAGAGATCGTCACTGCGG
GACTGATCGAAGAAGTCTACAACGTCAAAGCCTGTGCATCCCAGACCCCGTGAACAGCAAACCGATGATCGTGC
CACTGGAAAGATCTTAGGCAGCCGTGGGATTACACCCTTTTAGAGCTAGAACAGTAAAAATTCACCCAATAGCT
TTCAACTACGCACACAAAGTGGCAACATTGAGCGGGTGACTACAGACAAGCGCAAAACCTCTAAGACCACCGAC
ACCGCCAACAAGGCTGTGGGCGCGGATCAGGCAGCGCGTCCCACTCGGCGAACAACTCGCCGCATCTTCGATCA
GTCGGAGAAGATGAAGGACGTGCTGTACGAGATCCGTGGCCCGGTGGCCGCGGAGGCGGAACGCATGGAGCTTG
ATGGGCATAACATCTTAAAGCTCAACACGGGAAATCCAGCCGTGTTCGGATTCGATGCCCCCGACGTGATTATG
CGTGACATGATCGCCAACCTTCCAACTTCCCAAGGGTATTCCACCTCCAAAGGCATTATTCCGGCCCGGCGAGC
AGTGGTCACCCGCTACGAAGTTGTGCCCGGATTCCCCCACTTCGATGTTGATGATGTGTTCTTAGGCAACGGTG
TCTCAGAACTAATCACCATGACCACCCAAGCACTCCTCAACGACGGCGATGAAGTTCTTATCCCCGCACCGGAC
TACCCACTGTGGACTGCCGCAACCTCCCTGGCTGGTGGTAAGCCTGTGCACTACCTCTGTGATGAGGAAGATGA
CTGGAACCCATCCATCGAAGACATCAAGTCCAAAATCTCAGAGAAAACCAAAGCTATTGTGGTGATCAACCCCA
ACAACCCCACGGGAGCTGTCTACCCGCGCCGGGTGTTGGAACAAATCGTCGAGATTGCACGCGAGCATGACCTG
CTGATTTTGGCCGATGAAATCTACGACCGCATTCTCTACGATGATGCCGAGCACATCAGCCTGGCAACCCTTGC
ACCAGATCTCCTTTGCATCACATACAACGGTCTATCCAAGGCATACCGCGTCGCAGGATACCGAGCTGGCTGGA
TGGTATTGACTGGACCAAAGCAATACGCACGTGGATTTATTGAGGGCCTCGAACTCCTCGCAGGCACTCGACTC
TGCCCAAATGTCCCAGCTCAGCACGCTATTCAGGTAGCTCTCGGTGGACGCCAGTCCATCTACGACCTCACTGG
CGAACACGGCCGACTCCTGGAACAGCGCAACATGGCATGGACGAAACTCAACGAAATCCCAGGTGTCAGCTGTG
TGAAACCAATGGGAGCTCTATACGCGTTCCCCAAGCTCGACCCCAACGTGTACGAAATCCACGACGACACCCAA
CTCATGCTGGATCTTCTCCGTGCCGAGAAAATCCTCATGGTTCAGGGCACTGGCTTCAACTGGCCACATCACGA
TCACTTCCGAGTGGTCACCCTGCCATGGGCATCCCAGTTGGAAAACGCAATTGAGCGCCTGGGTAACTTCCTGT
CCACTTACAAGCAGTAG
SEQ ID No.8: Genome-integrated P9/P10 NCgl2747^{A955T}; and promoter sequence thereof (1645bp) 
CAAAGCACTTGCTACTCAATCATGGTAAACAGCCTGGTCGCAGTCCTTCACGATTCAAACTTTGCCTTCCGCTA
CGCCTTCCACCTGATCATCATAGAAGACGGTGAAGTAACAGCAGCCGGAGATCCCACAGAGATCGTCACTGCGG
GACTGATCGAAGAAGTCTACAACGTCAAAGCCTGTGCATCCCAGACCCCGTGAACAGCAAACCGATGATCGTGC
CACTGGAAAGATCTTAGGCAGCCGTGGGATTACACCCTTTTAGAGCTAGAACAGTAAAAATTCACCCAATAGCT
TTCAACTACGCACACAAAGTGGCAACATTGAGCGGGTGACTACAGACAAGCGCAAAACCTCTAAGACCACCGAC
ACCGCCAACAAGGCTGTGGGCGCGGATCAGGCAGCGCGTCCCACTCGGCGAACAACTCGCCGCATCTTCGATCA
GTCGGAGAAGATGAAGGACGTGCTGTACGAGATCCGTGGCCCGGTGGCCGCGGAGGCGGAACGCATGGAGCTTG
ATGGGCATAACATCTTAAAGCTCAACACGGGAAATCCAGCCGTGTTCGGATTCGATGCCCCCGACGTGATTATG
CGTGACATGATCGCCAACCTTCCAACTTCCCAAGGGTATTCCACCTCCAAAGGCATTATTCCGGCCCGGCGAGC
AGTGGTCACCCGCTACGAAGTTGTGCCCGGATTCCCCCACTTCGATGTTGATGATGTGTTCTTAGGCAACGGTG
TCTCAGAACTAATCACCATGACCACCCAAGCACTCCTCAACGACGGCGATGAAGTTCTTATCCCCGCACCGGAC
TACCCACTGTGGACTGCCGCAACCTCCCTGGCTGGTGGTAAGCCTGTGCACTACCTCTGTGATGAGGAAGATGA
CTGGAACCCATCCATCGAAGACATCAAGTCCAAAATCTCAGAGAAAACCAAAGCTATTGTGGTGATCAACCCCA
ACAACCCCACGGGAGCTGTCTACCCGCGCCGGGTGTTGGAACAAATCGTCGAGATTGCACGCGAGCATGACCTG
CTGATTTTGGCCGATGAAATCTACGACCGCATTCTCTACGATGATGCCGAGCACATCAGCCTGGCAACCCTTGC
ACCAGATCTCCTTTGCATCACATACAACGGTCTATCCAAGGCATACCGCGTCGCAGGATACCGAGCTGGCTGGA
TGGTATTGACTGGACCAAAGCAATACGCACGTGGATTTATTGAGGGCCTCGAACTCCTCGCAGGCACTCGACTC
TGCCCAAATGTCCCAGCTCAGCACGCTTTTCAGGTAGCTCTCGGTGGACGCCAGTCCATCTACGACCTCACTGG
CGAACACGGCCGACTCCTGGAACAGCGCAACATGGCATGGACGAAACTCAACGAAATCCCAGGTGTCAGCTGTG
TGAAACCAATGGGAGCTCTATACGCGTTCCCCAAGCTCGACCCCAACGTGTACGAAATCCACGACGACACCCAA
CTCATGCTGGATCTTCTCCGTGCCGAGAAAATCCTCATGGTTCAGGGCACTGGCTTCAACTGGCCACATCACGA
TCACTTCCGAGTGGTCACCCTGCCATGGGCATCCCAGTTGGAAAACGCAATTGAGCGCCTGGGTAACTTCCTGT
CCACTTACAAGCAGTAG
SEQ ID No.9: Downstream homologous arm sequence of genome-integrated P11/P12 NCgl2747 (596bp) 
CAGATGGCGCAATTAAATCAAGATCTCAGAACTCATTTTTCAATCTCTTCTTTTAGGGCACCCGTCATCAATTG
AGCTATCGGCCATTCATTAAAAACTGCGCGTCGATCAACCGAAAGCTGAGTAAGCAAAAAATTCGCCTCTTCTC
TATCGTCAAGAAGCAAAGCGCAACCAATCATAATCTCGCTATAGTTGTCTGGGTGCATTGTTTGATCAAGATTA
TGCCTAAAAGATCGAATTTCTGTTCTTTGTGAATCCAGTAATCCAGTTTGGCGATACAAAATTTGCCAACCATT
AAGCCTGTAAATTGGCGACTCTTCTCGATCTCGTTCCACGAGCCAATCATTGAGAGCTTGAGCTGCTATAAGAA
ATGCAGTTTTCCTGCTCTGCTCAGAATCAGCTGCCTTAATTAGGCGAAGCACCATCCATGTGGCGAGATTATCA
ACATCAACAGTTTCTTCAAGCGCTGAATAGAAATCAACAAGCTTATCCAAATGCAGATTCAATACTGATGGAAG
CCATCGATTATCAATGAGCTCGTACGGCGTCACTCGCTCGATTGAGGAGTCGCTCCGATCCGTTGAAGGTGTCA
TAGC
SEQ ID No.10: Identification primers P13 and P14 amplified fragment (size of 1959bp) 
TCCAAGGAAGATACACGCCCGAAGAAGCTCCGTTAATGGACGCTCTTCTATTAAGCCTTTTCTGATGGATACAA
GCAGAGAATCAGTCATGCTAAATATCATAATCCATCAAACATTTGATCGGTGTCAATCAATGCGTTCTGGACTG
AGGTGACCACCATGCAAGACGAGGTGGACCTAGTGATCACCAACCCGCCGTTTTCTCTGTTCCGTGAGTTCCTG
AGTTGGCTATTACACGGTGACGTGTTGTTTTCTATCATCGGTAACGCGAACGTAATCACATATCTAGGTGCCTG
AATCTAGATTAAAACTATAAACATTATTTAAAACCATTGCCTTATTGGAGCATGCTGCAAGCTTTTCGCGGTGG
GCTTGCAACATCTTACATCAGAATGAATGATGTTAAACACCTAATAAGTTCAAGTAGTTAAAAGGAAATATCAA
CAATGTTACGAAAAGCATCTATCACGCTAATGATATCCGTTACGCTCTTAACATGTGCCTCTCCAGCACAGGCA
CTGTCATCACAAGCACTCTCGTCAGAAAGCTCCACTTCGCAGAGTGATTCTCCTACGCAATTTGTTGCGAGTAT
AGCTGCGCCCTTCAATAAGAACTTAACCTATGAGCAGCGCAAAGCCATTCGTTTCGGACCTACGACAGAACAAG
AAGCAGAACAATGCTTGGCAAAGTATGGTCGCGATGGCGAGGGGCCATGGCCTGCTATTGCTATCTATCCTGAC
TGCTCCTTCGATACTATCGAACAAGATTTCTATGATCAAGCAATATCCAAAGCCCTCTCAGTAATCAGTCTGGG
AAGCTCCTAGAAGACTCGTAACGACCAAAAGCACCGAGAACAGATGCAACTCAAGCCCATTTACATCTGTTCTC
GGTGCACCAAAGCACTTGCTACTCAATCATGGTAAACAGCCTGGTCGCAGTCCTTCACGATTCAAACTTTGCCT
TCCGCTACGCCTTCCACCTGATCATCATAGAAGACGGTGAAGTAACAGCAGCCGGAGATCCCACAGAGATCGTC
ACTGCGGGACTGATCGAAGAAGTCTACAACGTCAAAGCCTGTGCATCCCAGACCCCGTGAACAGCAAACCGATG
ATCGTGCCACTGGAAAGATCTTAGGCAGCCGTGGGATTACACCCTTTTAGAGCTAGAACAGTAAAAATTCACCC
AATAGCTTTCAACTACGCACACAAAGTGGCAACATTGAGCGGGTGACTACAGACAAGCGCAAAACCTCTAAGAC
CACCGACACCGCCAACAAGGCTGTGGGCGCGGATCAGGCAGCGCGTCCCACTCGGCGAACAACTCGCCGCATCT
TCGATCAGTCGGAGAAGATGAAGGACGTGCTGTACGAGATCCGTGGCCCGGTGGCCGCGGAGGCGGAACGCATG
GAGCTTGATGGGCATAACATCTTAAAGCTCAACACGGGAAATCCAGCCGTGTTCGGATTCGATGCCCCCGACGT
GATTATGCGTGACATGATCGCCAACCTTCCAACTTCCCAAGGGTATTCCACCTCCAAAGGCATTATTCCGGCCC
GGCGAGCAGTGGTCACCCGCTACGAAGTTGTGCCCGGATTCCCCCACTTCGATGTTGATGATGTGTTCTTAGGC
AACGGTGTCTCAGAACTAATCACCATGACCACCCAAGCACTCCTCAACGACGGCGATGAAGTTCTTATCCCCGC
ACCGGACTACCCACTGTGGACTGCCGCAACCTCCCTGGCTGGTGGTAAGCCTGTGCACTACCTCTGTGATGAGG
AAGATGACTGGAACCCATCCATCGAAGACATCAAGTCCAAAATCTCAGAGAAAACCAAAGCTATTGTGGTGATC
AACCCCAACAACCCCACGGGAGCTGTCTACCCGCGCCGGGTGTTGGAACAAATCGTCGAGATTGCACGCGAGCA
TGACCTGCTGATTTTGGCCGATGAAATCTACGACC
SEQ ID No.11: Identification primers P15 and P16 amplified fragment (size of 1600bp) 
CAACGGTGTCTCAGAACTAATCACCATGACCACCCAAGCACTCCTCAACGACGGCGATGAAGTTCTTATCCCCG
CACCGGACTACCCACTGTGGACTGCCGCAACCTCCCTGGCTGGTGGTAAGCCTGTGCACTACCTCTGTGATGAG
GAAGATGACTGGAACCCATCCATCGAAGACATCAAGTCCAAAATCTCAGAGAAAACCAAAGCTATTGTGGTGAT
CAACCCCAACAACCCCACGGGAGCTGTCTACCCGCGCCGGGTGTTGGAACAAATCGTCGAGATTGCACGCGAGC
ATGACCTGCTGATTTTGGCCGATGAAATCTACGACCGCATTCTCTACGATGATGCCGAGCACATCAGCCTGGCA
ACCCTTGCACCAGATCTCCTTTGCATCACATACAACGGTCTATCCAAGGCATACCGCGTCGCAGGATACCGAGC
TGGCTGGATGGTATTGACTGGACCAAAGCAATACGCACGTGGATTTATTGAGGGCCTCGAACTCCTCGCAGGCA
CTCGACTCTGCCCAAATGTCCCAGCTCAGCACGCTATTCAGGTAGCTCTCGGTGGACGCCAGTCCATCTACGAC
CTCACTGGCGAACACGGCCGACTCCTGGAACAGCGCAACATGGCATGGACGAAACTCAACGAAATCCCAGGTGT
CAGCTGTGTGAAACCAATGGGAGCTCTATACGCGTTCCCCAAGCTCGACCCCAACGTGTACGAAATCCACGACG
ACACCCAACTCATGCTGGATCTTCTCCGTGCCGAGAAAATCCTCATGGTTCAGGGCACTGGCTTCAACTGGCCA
CATCACGATCACTTCCGAGTGGTCACCCTGCCATGGGCATCCCAGTTGGAAAACGCAATTGAGCGCCTGGGTAA
CTTCCTGTCCACTTACAAGCAGTAGCAGATGGCGCAATTAAATCAAGATCTCAGAACTCATTTTTCAATCTCTT
CTTTTAGGGCACCCGTCATCAATTGAGCTATCGGCCATTCATTAAAAACTGCGCGTCGATCAACCGAAAGCTGA
GTAAGCAAAAAATTCGCCTCTTCTCTATCGTCAAGAAGCAAAGCGCAACCAATCATAATCTCGCTATAGTTGTC
TGGGTGCATTGTTTGATCAAGATTATGCCTAAAAGATCGAATTTCTGTTCTTTGTGAATCCAGTAATCCAGTTT
GGCGATACAAAATTTGCCAACCATTAAGCCTGTAAATTGGCGACTCTTCTCGATCTCGTTCCACGAGCCAATCA
TTGAGAGCTTGAGCTGCTATAAGAAATGCAGTTTTCCTGCTCTGCTCAGAATCAGCTGCCTTAATTAGGCGAAG
CACCATCCATGTGGCGAGATTATCAACATCAACAGTTTCTTCAAGCGCTGAATAGAAATCAACAAGCTTATCCA
AATGCAGATTCAATACTGATGGAAGCCATCGATTATCAATGAGCTCGTACGGCGTCACTCGCTCGATTGAGGAG
TCGCTCCGATCCGTTGAAGGTGTCATAGCAAAATGATGACGCTTGGAAATTACTTCATGATAAGTCCACTCCCC
TGCAACATCACCAGGGTGAGCTATCAACGCAAGATTCCAACGACCA
SEQ ID No.12: NCgl2747 overexpressed by P17 and P18 amplified pXMJ19 plasmid and promoter sequence thereof (size of 1715bp) 
GCTTGCATGCCTGCAGGTCGACTCTAGAGGATCCCCCAAAGCACTTGCTACTCAATCATGGTAAACAGCCTGGT
CGCAGTCCTTCACGATTCAAACTTTGCCTTCCGCTACGCCTTCCACCTGATCATCATAGAAGACGGTGAAGTAA
CAGCAGCCGGAGATCCCACAGAGATCGTCACTGCGGGACTGATCGAAGAAGTCTACAACGTCAAAGCCTGTGCA
TCCCAGACCCCGTGAACAGCAAACCGATGATCGTGCCACTGGAAAGATCTTAGGCAGCCGTGGGATTACACCCT
TTTAGAGCTAGAACAGTAAAAATTCACCCAATAGCTTTCAACTACGCACACAAAGTGGCAACATTGAGCGGGTG
ACTACAGACAAGCGCAAAACCTCTAAGACCACCGACACCGCCAACAAGGCTGTGGGCGCGGATCAGGCAGCGCG
TCCCACTCGGCGAACAACTCGCCGCATCTTCGATCAGTCGGAGAAGATGAAGGACGTGCTGTACGAGATCCGTG
GCCCGGTGGCCGCGGAGGCGGAACGCATGGAGCTTGATGGGCATAACATCTTAAAGCTCAACACGGGAAATCCA
GCCGTGTTCGGATTCGATGCCCCCGACGTGATTATGCGTGACATGATCGCCAACCTTCCAACTTCCCAAGGGTA
TTCCACCTCCAAAGGCATTATTCCGGCCCGGCGAGCAGTGGTCACCCGCTACGAAGTTGTGCCCGGATTCCCCC
ACTTCGATGTTGATGATGTGTTCTTAGGCAACGGTGTCTCAGAACTAATCACCATGACCACCCAAGCACTCCTC
AACGACGGCGATGAAGTTCTTATCCCCGCACCGGACTACCCACTGTGGACTGCCGCAACCTCCCTGGCTGGTGG
TAAGCCTGTGCACTACCTCTGTGATGAGGAAGATGACTGGAACCCATCCATCGAAGACATCAAGTCCAAAATCT
CAGAGAAAACCAAAGCTATTGTGGTGATCAACCCCAACAACCCCACGGGAGCTGTCTACCCGCGCCGGGTGTTG
GAACAAATCGTCGAGATTGCACGCGAGCATGACCTGCTGATTTTGGCCGATGAAATCTACGACCGCATTCTCTA
CGATGATGCCGAGCACATCAGCCTGGCAACCCTTGCACCAGATCTCCTTTGCATCACATACAACGGTCTATCCA
AGGCATACCGCGTCGCAGGATACCGAGCTGGCTGGATGGTATTGACTGGACCAAAGCAATACGCACGTGGATTT
ATTGAGGGCCTCGAACTCCTCGCAGGCACTCGACTCTGCCCAAATGTCCCAGCTCAGCACGCTATTCAGGTAGC
TCTCGGTGGACGCCAGTCCATCTACGACCTCACTGGCGAACACGGCCGACTCCTGGAACAGCGCAACATGGCAT
GGACGAAACTCAACGAAATCCCAGGTGTCAGCTGTGTGAAACCAATGGGAGCTCTATACGCGTTCCCCAAGCTC
GACCCCAACGTGTACGAAATCCACGACGACACCCAACTCATGCTGGATCTTCTCCGTGCCGAGAAAATCCTCAT
GGTTCAGGGCACTGGCTTCAACTGGCCACATCACGATCACTTCCGAGTGGTCACCCTGCCATGGGCATCCCAGT
TGGAAAACGCAATTGAGCGCCTGGGTAACTTCCTGTCCACTTACAAGCAGTAGGTTTTGGCGGATGAGAGAAGA
TTTTCAGCCTGAT
SEQ ID No.13: NCgl2747^{A955T}; overexpressed by P17 and P18 amplified pXMJ19 plasmid and promoter sequence thereof (size of 1715bp) 
GCTTGCATGCCTGCAGGTCGACTCTAGAGGATCCCCCAAAGCACTTGCTACTCAATCATGGTAAACAGCCTGGT
CGCAGTCCTTCACGATTCAAACTTTGCCTTCCGCTACGCCTTCCACCTGATCATCATAGAAGACGGTGAAGTAA
CAGCAGCCGGAGATCCCACAGAGATCGTCACTGCGGGACTGATCGAAGAAGTCTACAACGTCAAAGCCTGTGCA
TCCCAGACCCCGTGAACAGCAAACCGATGATCGTGCCACTGGAAAGATCTTAGGCAGCCGTGGGATTACACCCT
TTTAGAGCTAGAACAGTAAAAATTCACCCAATAGCTTTCAACTACGCACACAAAGTGGCAACATTGAGCGGGTG
ACTACAGACAAGCGCAAAACCTCTAAGACCACCGACACCGCCAACAAGGCTGTGGGCGCGGATCAGGCAGCGCG
TCCCACTCGGCGAACAACTCGCCGCATCTTCGATCAGTCGGAGAAGATGAAGGACGTGCTGTACGAGATCCGTG
GCCCGGTGGCCGCGGAGGCGGAACGCATGGAGCTTGATGGGCATAACATCTTAAAGCTCAACACGGGAAATCCA
GCCGTGTTCGGATTCGATGCCCCCGACGTGATTATGCGTGACATGATCGCCAACCTTCCAACTTCCCAAGGGTA
TTCCACCTCCAAAGGCATTATTCCGGCCCGGCGAGCAGTGGTCACCCGCTACGAAGTTGTGCCCGGATTCCCCC
ACTTCGATGTTGATGATGTGTTCTTAGGCAACGGTGTCTCAGAACTAATCACCATGACCACCCAAGCACTCCTC
AACGACGGCGATGAAGTTCTTATCCCCGCACCGGACTACCCACTGTGGACTGCCGCAACCTCCCTGGCTGGTGG
TAAGCCTGTGCACTACCTCTGTGATGAGGAAGATGACTGGAACCCATCCATCGAAGACATCAAGTCCAAAATCT
CAGAGAAAACCAAAGCTATTGTGGTGATCAACCCCAACAACCCCACGGGAGCTGTCTACCCGCGCCGGGTGTTG
GAACAAATCGTCGAGATTGCACGCGAGCATGACCTGCTGATTTTGGCCGATGAAATCTACGACCGCATTCTCTA
CGATGATGCCGAGCACATCAGCCTGGCAACCCTTGCACCAGATCTCCTTTGCATCACATACAACGGTCTATCCA
AGGCATACCGCGTCGCAGGATACCGAGCTGGCTGGATGGTATTGACTGGACCAAAGCAATACGCACGTGGATTT
ATTGAGGGCCTCGAACTCCTCGCAGGCACTCGACTCTGCCCAAATGTCCCAGCTCAGCACGCTTTTCAGGTAGC
TCTCGGTGGACGCCAGTCCATCTACGACCTCACTGGCGAACACGGCCGACTCCTGGAACAGCGCAACATGGCAT
GGACGAAACTCAACGAAATCCCAGGTGTCAGCTGTGTGAAACCAATGGGAGCTCTATACGCGTTCCCCAAGCTC
GACCCCAACGTGTACGAAATCCACGACGACACCCAACTCATGCTGGATCTTCTCCGTGCCGAGAAAATCCTCAT
GGTTCAGGGCACTGGCTTCAACTGGCCACATCACGATCACTTCCGAGTGGTCACCCTGCCATGGGCATCCCAGT
TGGAAAACGCAATTGAGCGCCTGGGTAACTTCCTGTCCACTTACAAGCAGTAGGTTTTGGCGGATGAGAGAAGA
TTTTCAGCCTGAT
SEQ ID No.14: Homologous arm sequence of knockout NCgl2747(size of 1400bp)

GCAGCAAGTAGCCTGGGTATTCGCCACGGACGTGAATATTGCCGAAGGCATAGTCCTCGTGGGACTTTTGCTGG
GCGGTAAGTTGATCGCGTGGGTCCGGGGTAATGCCATAACGAGGAACGGCAATAATCATGCAACCGATCTGGTT
TTGTGGGTCGATCTCATGAGCAATCTTAGTTGCCAAAGCACTTGCTACTCAATCATGGTAAACAGCCTGGTCGC
AGTCCTTCACGATTCAAACTTTGCCTTCCGCTACGCCTTCCACCTGATCATCATAGAAGACGGTGAAGTAACAG
CAGCCGGAGATCCCACAGAGATCGTCACTGCGGGACTGATCGAAGAAGTCTACAACGTCAAAGCCTGTGCATCC
CAGACCCCGTGAACAGCAAACCGATGATCGTGCCACTGGAAAGATCTTAGGCAGCCGTGGGATTACACCCTTTT
AGAGCTAGAACAGTAAAAATTCACCCAATAGCTTTCAACTACGCACACAAAGTGGCAACATTGAGCGGGTGACT
ACAGACAAGCGCAAAACCTCTAAGACCACCGACACCGCCAACAAGGCTGTGGGCGCGGATCAGGCAGCGCGTCC
CACTCGGCGAACAACTCGCCGCATCTTCGATCAGTCGGAGAAGATGAAGGACGTGGGTAACTTCCTGTCCACTT
ACAAGCAGTAGTAGTTGTTAGGATTCACCACGAATCTCAGGATTTTTGAGATTCGTGGTGAATTTTTGCGTTTT
CCAGTCAGGCTCCTGCAACTTTCGGACCGATTTCAGAGGGGCGGAGCTGGTTTGTGGTGGATCCTTGAAATGGA
ACCTCGCAGGAAGCTTTCAGGAAGACCAAGTTGGGCCTAGGGGTGGCGGGATTGCAAAAATCCGTCCCCGGTTC
GCCATGAAATGCTGATTTTGATCGAATCTTTGCGCTAACTGTAGGGCGGGTTCAGGGGGTGAATGCACCACGAG
CAACCCGAAGGGTGCGAAGTGGGCATTCGTAGAACAATCCCAGAGGAAAGCCGTACGGCTTTCCTCGACATGAT
CAATCAAGGTATGTCAGGTCTTGCTGCGTCTACAGCGGTCGGGGTCAGTGAATTCACCGGGCGAAAGTGGGCGA
AGGCCGCCGGGGTGAAACTGACCCGCGGCCCGCGAGGTGGCAATGCTTTTGACACCGCCGAGAAACTTGAGATT
GCAGCCAGCATGCTAGAGAAAGGATGCCTACCCCGAGAAATCGGCGAGTATGTCGGCATGACTCGGGCCAATAT
ATCCCTATGGCGCAAACAAGGCCCAGACAAGCTTCGCCAACGCGCAGCCACCTTGCGCACCGGCAAGCGAGCAG
CTGAATTCATCCACGCCCCGGTGATGGGCCCTTATTATGGGCCACGCACACTCCATCAAGTGTTGCGT

### Industrial application

According to the present invention, point mutation NCgl2747^{I319F} and overexpression of the NCgl2747 gene coding region in the *Corynebacterium glutamicum* contributes to increase in the yield and the growth rate of L-lysine. However, when the gene is weakened or knocked out, accumulation of the L-lysine is not facilitated, and the growth rate of a strain can be reduced. It shows that NCgl2747^{I319F} and its encoding gene of the present invention can be used to prepare the L-lysine and have good application prospects.

## Claims

1. A protein, which is the following A1) or A2):
A1) a protein comprising an amino acid sequence of SEQ ID No.4;
A2) a fusion protein obtained by connecting a tag to an N-terminal or/and a C-terminal of A1).

2. A biological material related to the protein according to claim 1, which is any one of the following B1) to B4):
B1) a nucleic acid molecule encoding the protein according to claim 1;
B2) an expression cassette comprising the nucleic acid molecule in B1);
B3) a recombinant vector comprising the nucleic acid molecule in B1), or a recombinant vector comprising the expression cassette in B2);
B4) a recombinant microorganism comprising the nucleic acid molecule in B1), or a recombinant microorganism comprising the expression cassette in B2), or a recombinant microorganism comprising the recombinant vector in B3).

3. The biological material according to claim 2, wherein the nucleic acid molecule in B1) is the following b11) or b12) or b13):
b11) a DNA molecule shown in SEQ ID No.3 in a sequence listing;
b12) a DNA molecule that has 75% or more identity with a nucleotide sequence defined by b11) and is used for encoding the protein according to claim 1;
b13) a genomic DNA molecule that hybridizes with the nucleotide sequence defined by b11) or
b12) under stringent conditions and is used for encoding the protein according to claim 1.

4. The biological material according to claim 2 or 3, wherein the expression cassette in B2) is the DNA molecule shown in SEQ ID No.8;
the recombinant microorganism in B4) is a recombinant microorganism obtained by replacing the NCgl2747 gene in the microorganism containing the NCgl2747 gene shown in SEQ ID No.1 with the nucleic acid molecule in B1), or introducing the nucleic acid molecule in B1) into the microorganism and expressing the nucleic acid molecule.

5. A method for preparing L-lysine, comprising: expressing the protein according to claim 1 in a recipient biological cell, or increasing the content or activity of the protein according to claim 1 in the recipient biological cell, or increasing the content or activity of the protein shown in SEQ ID No.2 in the recipient biological cell to obtain a recombinant biological cell; and culturing the recombinant biological cell to obtain the L-lysine.

6. The method according to claim 5, wherein the biological cell is yeast, bacterium, algae, fungi, a plant cell or an animal cell capable of synthesizing the L-lysine.

7. The method according to claim 6, wherein the bacterium is *Corynebacterium glutamicum.*

8. The method according to any one of claims 5-7, wherein the method is realized by introducing an encoding gene of the protein according to claim 1 into the recipient biological cell and expressing the encoding gene, or introducing an encoding gene of the protein shown in SEQ ID No.2 into the recipient biological cell and expressing the encoding gene;
or, the recipient biological cell contains a DNA molecule shown in SEQ ID No.1, and the method is realized by replacing the DNA molecule shown in SEQ ID No.1 in the recipient biological cell with a DNA molecule shown in SEQ ID No.3.

9. The method according to any one of claims 5-7, wherein the recombinant biological cell is cultured using a culture medium capable of growing the recombinant biological cell;
and/or, the recombinant biological cell is cultured using conditions that enable the growth of the recombinant biological cell.

10. The method according to claim 9, wherein the method is realized by introducing an encoding gene of the protein according to claim 1 into the recipient biological cell and expressing the encoding gene, or introducing an encoding gene of the protein shown in SEQ ID No.2 into the recipient biological cell and expressing the encoding gene;
or, the recipient biological cell contains a DNA molecule shown in SEQ ID No.1, and the method is realized by replacing the DNA molecule shown in SEQ ID No.1 in the recipient biological cell with a DNA molecule shown in SEQ ID No.3.

11. A product for preparing L-lysine, comprising the protein according to claim 1 or the biological material according to any one of claims 2-4.
